# EUROPEAN PATENT APPLICATION

(11) **EP 4 039 216 A1**
(43) Date of publication of application: **10.08.2022**
(21) Application number: 21155596.6
(22) Date of filing: 05.02.2021
(51) Int. Cl.: A61B 34/30, A61B 90/50, A61B 90/00

(54) **INSTRUMENT HOLDER**

(71) Applicant: Metal Aarschot NV, 3200 Aarschot (BE)
(72) Inventor: Thijs, Tom, 3200 Aarschot (BE); Thijs, André, 3200 Aarschot (BE); Thijs, Andy, 3200 Aarschot (BE)
(74) Representative: Arnold & Siedsma

(57) **Abstract**

The present invention is directed to a holder which has to be combined with an external object whereby the external object moves around a remote centre of motion. The external object preferably is a medical instrument such as a cannula or trocar whereby the remote centre of motion is the patient's abdomen. The holder consists of two members, generating motion in both a longitudinal and rotational manner.

The holder can be used to support another object, in a particular embodiment a camera, convenient for performing endoscopic (laparoscopic) surgery and is equipped with means for generating motion, both in a longitudinal and rotational manner following XYZ coordinates. The holder of the present invention is particularly useful for working in small areas as it occupies only a small bedside space, without scarifying on functional work area of the instrument mounted in the holder.

## Description

### FIELD OF THE INVENTION

The present invention is directed to a holder to be combined with an external object whereby the external object moves around a centre of motion. This centre of motion lays remote fo the holder. The external object preferably is a medical instrument such as a cannula or trocar whereby the centre of motion is the incision in the patient's abdomen. The holder can be used to support another object, in a particular embodiment a camera, convenient for performing endoscopic surgery, including in video-assisted thoracoscopic surgery, laparoscopic surgery and arthroscopic surgery; and is equipped with means for allowing motion of the object, both in a longitudinal and rotational manner following XYZ coordinates. The holder of the present invention is particularly useful for working in small areas as it occupies only a small bedside space, without sacrificing on the functional work area of the instrument mounted in the holder.

### BACKGROUND TO THE INVENTION

In standard laparoscopic abdominal surgery, incisions are made into the abdomen, followed by insufflation of the patient's abdomen with gas, and passing through cannulas via the small (approximately 1/2 inch) incisions to provide entry ports for laparoscopic surgical instruments. The laparoscopic surgical instruments generally include a laparoscope for viewing the surgical field, and working tools such as clamps, graspers, scissors, staplers, and needle holders. The working tools are similar to those used in conventional (open) surgery, except that the working tools are often thin and long with at one end tools working in the surgical field and at the other end handles manipulated by a surgeon. To perform surgical procedures, the surgeon passes instruments through the cannulas and manipulates them inside the abdomen by sliding them in and out through the cannulas, rotating them in the cannulas, and "levering" (pivoting) them around the centres of rotation approximately defined by the incisions in the muscles of the abdominal wall. This point is generally referred to as the Remote Centre of Motion (RCM). To maintain accurate positional control of an instrument during surgery, the surgeon may need to manually constrain it to pivot around the RCM coincident with the incision. Manual support of the pivot point is particularly of importance when the surgeon employs laparoscopes or other heavy instruments. Mechanical clamping devices are used to support the instruments in fixed orientations, but these devices do not provide a remote centre of rotation for positioning the instruments around the RCM.

Such instrument holder has been described in EP3294187 enabling positioning of the instrument holder around a RCM. In said system, het holder comprises a first member, a second member and a coupling means. The first member forms the basis and is configured to allow both a longitudinal and rotational movement of the second member along its longitudinal axis. A coupling means, able to be coupled to the object, is connected to the first member via a second member. In certain embodiments described in EP3294187 the second member is formed by two arms, connected to guides at the first member enabling the longitudinal displacement of second members along its longitudinal axis. An advantage of using two arms as second member is that both the absolute and the rotational position of the coupling means can be fixated via the two arms by locking further movements of the first and second members. Further locking means at the coupling means, to lock movement of the coupling means with respect to the second member, is unnecessary. A drawback of this instrument holder relates to the freedom a user has in moving the external object. The longitudinal displacement of the two arms is predetermined so that the freedom of movement is significantly limited.

It is accordingly an objective of the present invention to improve the flexibility and usability of the instrument holder.

### SUMMARY OF THE INVENTION

In a first aspect, the present invention provides a holder having a first member oriented in line with an axis with its origin at a remote centre of motion. The holder further has a second member comprising two arms, each arm having a first end connected by means of a pivotable connector to the first member, the pivotable connectors allowing the arms to pivot about a further axis perpendicular to said axis. The holder has coupling means for holding said external object, the coupling means being pivotally connected to a second end of each arm. The first member is configured to allow a longitudinal and circumferential displacement of the pivotable connector along said axis of the first member.

By connecting the two arms to the first member via pivotable connectors allowing the arms to pivot about a further axis perpendicular to the axis, a further degree of freedom of movement of the coupling means is obtained. The two arms preferably extend in a plane comprising or at least parallel to said axis , and the further axis is perpendicular to that plane. By pivoting the arms about the pivotable connectors, the arms can make a rotational movement in the plane. By longitudinally displacing the pivotable connectors along the axis of the first member, the arms can make a translational movement in the plane. The plane itself, including the arms, can rotate about the axis when the first member is circumferentially displaced. These combinations of movements allow a user to place and orient the coupling means, including the external object held by it, in a wide range of positions. The holder of the invention thereby improves the flexibility and usability of the instrument holder.

Preferably the first member comprises a guide for each arm to allow a longitudinal displacement of the pivotable connections along said axis of the first member. The guides define the trajectory that each one of the pivotable connections can follow in the first member. The guides are independently formed allowing independent movement of each arm. The skilled person will understand that since the second ends of the arms are connected to the coupling means, uneven movement of the arms in the guides will induce a rotation of the coupling means. Via the guide, the movement of the pivotable connections can be controlled. Furthermore, via the guide, locking of the longitudinal movement of the pivotable connections can be embodied. Preferably the guide is selected from a linear guide and a curved guide. The type of guide determines how the user experiences and is assisted in the movement.

Preferably an abutment is provided which forms a touch edge for one of the arms of the second member. Preferably the abutment is positioned on the first member proximal to the remote centre of motion of said object. The abutment defines a lowest rotational position of the second member. The abutment assists the user in carrying the external object. As explained above, objects such as laparoscopes or other heavy instruments are used by surgeons, and are positioned with respect to a centre of motion which is remote for the holder. The abutment prevents these objects from descending below a predetermined point.

Preferably the abutment is formed by a cam roller acting upon the second member. Preferably the cam of the cam roller is exchangeable. Providing a cam roller, which is typically a non-round or eccentric roller, determines the trajectory of the arms when the arms are moved in the longitudinal direction while the pivotable connections are down. Lifting up the arms by rotating the pivotable connections upward will result in a distance between the abutment and the arms. This clarifies that the arms can move freely but are assisted and supported in a predetermined lower movement.

Preferably at least one of the arms, preferably both arms of the second member comprises a curved section at the end where it is connected to the first member. Preferably said coupling means are displaced offset from said axis by means of said second member.

Preferably the longitudinal displacement of the pivotable connectors along said axis of the first member is realized by adapting a slidable position of the corresponding pivotable connectors at the first member.

Preferably the circumferential displacement of the pivotable connectors along said axis of the first member is realized by rotation of the first member along its longitudinal axis.

Preferably the external object is a medical instrument or medical tool (e.g. cannula, trocar, ...).

Preferably said holder further comprises locking means for locking the pivotable connectors and the longitudinal and circumferential displacement to fixate the position of the coupling means relative to the first member. Via the locking means, the position of the coupling means can be fixated thereby fixating the external object held by it. As explained above, the holder of the invention enables to fixate both the absolute and rotational position of the external object.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

In the figures:
figure 1 shows a cross section of an instrument holder of an embodiment of the invention;
figure 2 shows a cross section of a segment of an instrument holder according to an embodiment;
figure 3 shows a cross section of a segment of a further embodiment;
figure 4 shows a cross section of a segment of an instrument holder according to another further embodiment; and
figure 5 shows a three-dimensional view of an embodiment of an instrument holder.

### DETAILED DESCRIPTION OF THE INVENTION

In general, the goal of the present invention is to provide an instrument holder for an object, in a particular embodiment a camera, during laparoscopic surgery or other high precision surgeries, i.e. minimal invasive surgical procedures such as video-assisted thoracoscopic surgery, and arthroscopic surgery, that has to be combined with another object, in a particular embodiment a cannula or trocar that moves around a centre of motion, preferably being the incision in the patient, for example the incision in the patient's abdomen in case of laparoscopic surgery. Expressed differently, the external object is held at an isocenter, being its centre of motion, and is connected to the holder wherein the holder is configured to follow the motion of the external object around the isocenter, being around a remote centre of motion RCM. The RCM may be a fixed or non-fixed RCM. It will be clear from this explanation that the centre of motion of the external object is a remote centre of motion for the holder.

The present invention provides a holder for an object, preferably an external object, in a particular embodiment a cannula or trocar for minimally invasive surgical procedures, said holder having two members. The instrument holder of the instant application can be used during a minimally invasive surgical procedure selected from video-assisted thoracoscopic surgery, laparoscopic surgery, and arthroscopic surgery; more in particular laparoscopic surgery. In said last embodiment the remote centre of motion coincides with the incision in the patient's abdomen.

Not being limited to laparoscopic surgery, various tools or instruments like cannulas, trocars and medical instruments having a centre of motion, like the narrow entry points in laparoscopic surgery or other high precision surgery, can be supported by this invention. In said embodiment wherein the object is a cannula or trocar, various tools, like a laparoscope can be inserted into said cannula or trocar and accordingly held by the holder of the instant application. These various tools or instruments are named the external object 300 in the description hereunder. Alternatively, the medical instrument itself, such as laparoscope, forms the external object.

For this initial orientation of the first member along said axis 1, the holder is typically mounted in a tripod or mounting fixture in the proximity of the isocenter or remote centre of motion 8.

As will be apparent to the skilled artisan, the holder can be used passively, wherein the movement of the holder passively follows the movements of the external object when it is manipulated by a user. Alternatively, the holder is used actively and controls the movement of the external object. Accordingly, in a further embodiment to the invention, the device comprises means for generating motion, both in a longitudinal and rotational manner following XYZ coordinates, thereby holding a given object, such as a camera.

Preferably, the holder is made of sustainable materials, which in a certain embodiment are heat stable and therefore suitable for heat sterilisation.

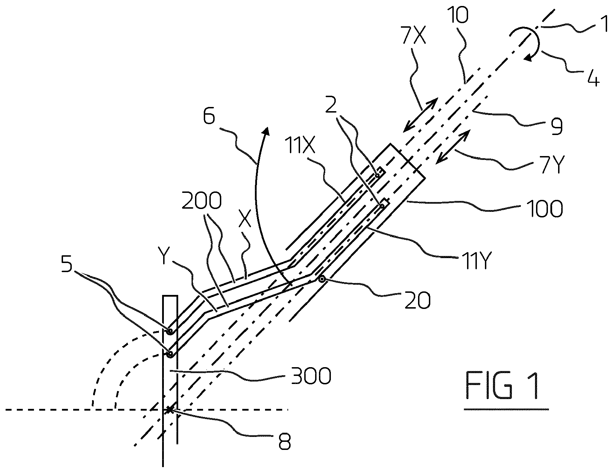

Figure 1 shows an instrument holder having multiple parts. The instrument holder has a first member 100 and a second member 200. The second member 200 comprises two arms X and Y. Via the first member 100 and the second member 200, an external object 300 can be held. This external object is considered to be the instrument held by the instrument holder. In the embodiment of figure 1, the external object 300 is connected to the second member 200 via coupling means 5.

The first member 100 and the second member 200 operate together to allow the external object 300 to rotate about a remote centre of motion 8. The remote centre of motion is remote to the instrument holder. This remote centre of motion 8 is typically located somewhere along the external object 300. As described above, this allows the external object 300 to pass through an incision made in the body of a patient, and allows the external object to be moved and re-oriented keeping the location where the external object passes through the incision stable. The instrument holder with the first member 100 and the second member 200 is intended to facilitate moving an re-orienting the external object 300 with respect to the remote centre of motion 8 as well as to allow a large freedom of motion. Furthermore, it is beneficial when the instrument holder can be locked after the external object 300 has been positioned. In the locked state, the external object 300 is held stable in the chosen position and orientation.

The first member 100 is formed as a housing extending along an axis 1. In use of the instrument holder the first member 100 is oriented with respect to the remote centre of motion 8 such that the remote centre of motion 8 is located on the axis 1. The advantage of this is that a rotational movement 4 of the first member 100 along its axis 1 induces a corresponding movement of the external object 300 around the remote centre of motion 8.

The housing of the first member 100 is provided with a first guide 11X and a second guide 11Y. The guides extend along second axis 10 and third axis 9, both being parallel to the axis 1. The first guide 11X is formed to allow a longitudinal movement 7X of a first arm X of the second member 200 along the second axis 10. The second guide 11Y allows a longitudinal movement 7Y of a second arm Y of the second member 200 along the third axis 9. Each of the guides 11X and 11Y extends over at least 50% of the length of the first member 100, preferably over at least 60% of the length of the first member 100, more preferably of at least 70% of the length of the first member. This allows a substantial movement of the arms in the longitudinal direction such that the instrument holder can be made compact while allowing a significant freedom of motion for the external object 300.

The second member 200 comprises two arms X and Y. The two arms X and Y can be shaped identical or substantially identical or can be shaped slightly different depending on the intended use. The two arms X and Y interconnect the first member 100 and the external object 300. The two arms X and Y are connected to the first member 100 centred to the axis 1. This means that the first arm X is connected to the first member 100 at a first location and the second arm Y is connected to the first member 100 at a second location and that a third location defined as the middle between the first location and the second location lays on the axis 1. At the external object 300, the arms X and Y are connected offset from the axis 1. This means that the arms X and Y connected to the external object at a distance from the axis 1 and are both connected at the same side of the axis 1.

Each of the arms X and Y has a first segment, connecting the arm to the first member 100, the first segment extending substantially parallel to the first member 100. Each of the arms X and Y further has a second segment where the arm is connected to the external object. Between the first segment and the second segment each arm has an intermediate segment extending at an angle with respect to the axis 1. This angle is preferably larger than 20°, more preferably larger than 30°, this angle is preferably smaller than 90°, more preferably smaller than 60°. Via the intermediate section, the arms X and Y bridge the distance between the centred connection to the first member and the offset connection to the external object 300.

Each arm X and Y is pivotably connected to the external object 300 via coupling means 5 at a first end of the arm, and is pivotably connected to the first member 100 via pivotable connector 2 and at a second end of the arm. The two arms thereby form a parallelogram mechanism, the behaviour of which is known to the skilled person. In the context of the instrument holder, the parallelogram mechanism allows to lock the position and orientation of the external object 300 by locking movement of the first member and the arms X and Y at the first member only. There is no need to lock the coupling means 5 or to provide an additional locking mechanism at the instrument holder. This is an advantage since it simplifies the instrument holder and the use thereof.

The pivotable connectors 2 of the arms X and Y are connected to respective guides 11X and 11Y. This allows the pivotable connectors 2 to make a longitudinal movement 7X and 7Y along the first member 100. Furthermore, the arms X and Y can make a pivoting movement 6 with respect to the pivotable connector 2, providing a significant freedom of motion to the external object 300. Furthermore, the pivotable connectors 2 are preferably individually moveable in the guides 11X and 11Y. Because the arms X and Y operate as a parallelogram mechanism, making a different longitudinal movement 7 will induce a rotation of the external object 300 in a plane defined by the external object 300 and the axis 1. Rotation of the first member 100, indicated with arrow 4, will induce a rotation of the external object 300 in a plane perpendicular to the axis 1. Combinations of these rotations allows the instrument holder to freely rotate the external object 300 with respect to the remote centre of motion 8.

In figure 1, the coupling means 5 are simply shown as a direct pivotable connection between the external object 300 and the arms X and Y. alternatively, the coupling means can be formed as a connector element or clamping element to which the external object 300 is connected or clamped. In a particular embodiment the flexible coupling is exchangeable. In this embodiment the second member comprises an adaptor, enabling said flexible coupling to be placed at the free end of the second member.

Figure 1 further shows an abutment 20 for the second arm Y. As described above, the arms are connected to the first member 100 via pivotable connectors 2. The abutment 20 defines a lowest rotational position of the second arm Y. Via the abutment 20, it can be prevented that the external object 300 drops down below a predetermined point.

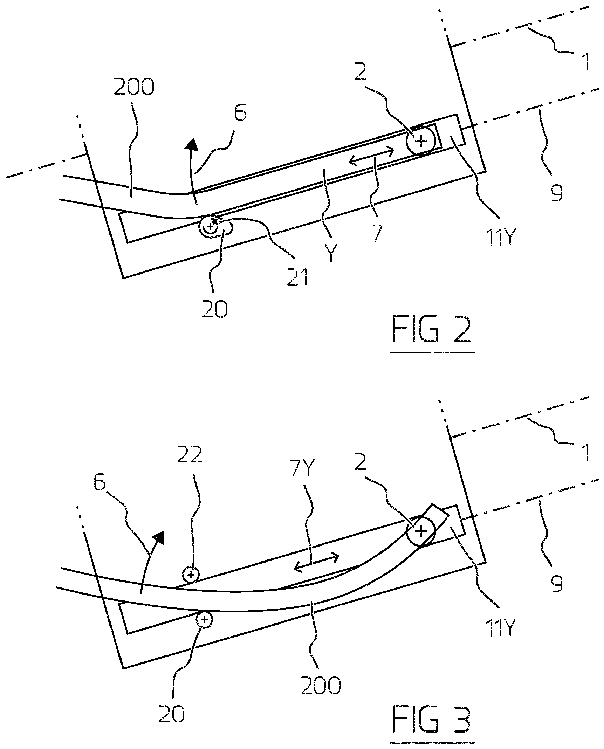

Figure 2 shows an abutment 20 in the form of a ridge. Rotation of the abutment 21, for example due to a longitudinal movement 7 of the arm Y induces a pivoting movement 6. The shape and size of the ridge 20 determines the upward and downward pivoting movement 6 that is induced on the second arm Y. Preferably, the abutment 20 is interchangeable. This allows a user of the instrument holder to select an abutment 20 depending on the desired behaviour of the arm Y when the arm Y is longitudinally moved 7. This abutment 20, particularly when a ridge is selected in correspondence with the external object 300, significantly facilitates repositioning the external object 300 with respect to the remote centre of motion 8. Particularly, when the external object 300 has a non-negligible weight, it will be beneficial that the abutment 20 controls the lowest position of the external object in function of the longitudinal position 7. This enables the instrument holder to force the external object to follow a predetermined path as is indicated in figure 1 in dotted lines on the left hand side of the figure.

Figure 3 shows an alternative embodiment wherein the abutment 20 is circular and wherein the first segment of the arm Y is shaped to induce this pivoting movement 6. Due to the non-straight shape of the arm, a longitudinal movement 7Y will force the arm into an upward and downward pivoting movement 6. In figure 3, a counter abutment 22 is shown preventing the arm to make a pivoting movement 6 upward beyond the counter abutment.

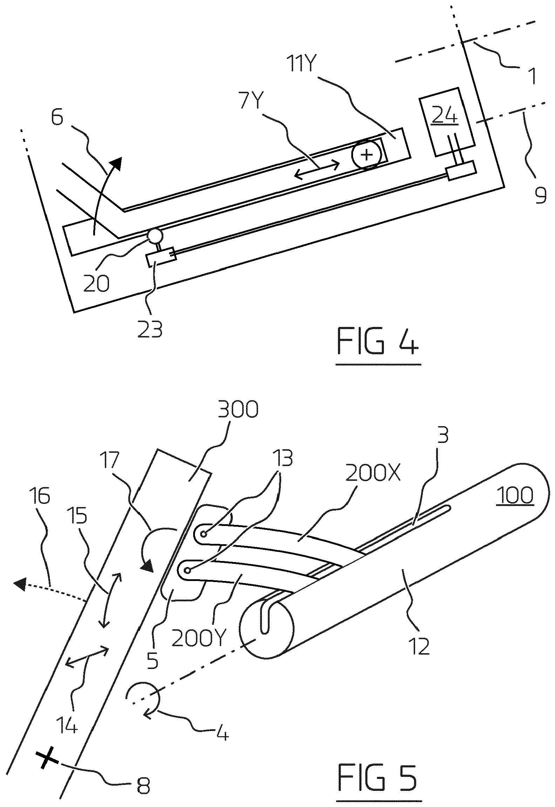

Figure 4 shows yet another embodiment wherein the abutment 20 is mounted on a height control mechanism 23 to control the position of the abutment 20. This height control mechanism 23 is preferably connected to an actuator 24 to control the position of the abutment 20 with respect to the guide 11. Via the actuator, the pivoting movement 6 can be controlled independently from the longitudinal movement 7Y.

Figure 5 shows a perspective view of an instrument holder holding an external object 300. Figure 5 shows the housing 12. The housing 12 is provided with a slit 3, allowing the arms 200X and 200Y to extend between an inside and an outside of the housing 12. The slit 3 is preferably located at least partially in a side surface of the first member. The side surface is the surface extending substantially parallel to the axis 1. In figure 5, the slit extends from the side surface into the distal surface into the first member 100. The arms X and Y are connected via the pivotable connectors 2 to the guides 11 inside the housing 12. The mechanism inside the housing 12 is shown in and explained with respect to figures 1-4.

In figure 5, the coupling means 5 are provided as a separate element to which the external object 300 can be connected. The coupling means are pivotably connected 13 to the arms 200X and 200Y.

From the explanation above, it will be clear that the external object 300 can make a first translational movement 14 by longitudinally moving 7 the pivotable connector 2 along the guides 11. The external object 300 can make a second translational movement 15 by pivoting the arms X and Y around the pivotable connector 2. The external object 300 can make a pivotable movement 17 by differently moving the arms X and Y in the guides 11. Finally, the external object can make a rotational movement 4 by rotating the first member 100 along the axis 1. Combination of these movements and rotations allows the external object 300 to make a preferred movement 16 allowing a movement and repositioning of the external object 300 with respect to the remote centre of motion 8.

In the first member 100, locking means are provided to lock further longitudinal movement 7 of the arms with respect to the guide. This locks the first translational movement 14 and locks the pivotable movement 17 due to the parallelogram mechanism. Furthermore, the first member 100 comprises locking means to lock the pivoting movement 6 of the arms with respect to pivotable connector 2, thereby locking the second translational movement 15. Finally, the first member 100 preferably additionally comprises a locking mechanism for locking rotation 4 around the axis. Combinations of these locking mechanisms allow to hold to external object 300 in a stable and predetermined position. Furthermore, unlocking of these locking means allow a user to freely move the external object with respect to the remote centre of motion 8. Via the abutment 20, the user is assisted in moving the external object 300 and dropping of the external object 300 prevented. The locking means can be formed by friction couplings. The friction couplings are preferably operable using a manipulator. In a particular embodiment, the manipulator uses air pressure to lock the friction couplings.

To summarize, the present invention provides a holder, particularly useful as an instrument, preferably camera, holder in minimally invasive surgical procedures and differs from the current RCM manipulators in that the holder in itself does not need to have an RCM. In a preferred embodiment this external object is a trocar for laparoscopic or video-assisted thoracoscopic surgery and inserted into the incision of the patient's abdomen or thorax. When inserted this trocar is spatially constraint at said incision point, making this point to behave as a centre of motion for the trocar. Alternatively, the external object is the laparoscopic instrument itself. Coupling the object to the holder of the instant application does not further constrain the manipulation of the object. The holder accordingly provides a compact solution to keep a laparoscope or endoscope at a desired orientation, without making concessions on the operational area of manipulation. This large operational area of manipulation is a significant advantage for example during laparoscopic surgery.

## Claims

1. A holder
- having a first member (100) oriented in line with an axis (1) with its origin at a remote centre of motion (8);
- having a second member (200) comprising two arms (X) and (Y), each arm having a first end connected by means of a pivotable connector (2X, 2Y) to the first member (100), the pivotable connectors allowing the arms to pivot about a further axis perpendicular to said axis (1);
- having coupling means (5X, 5Y) for holding said external object (300), the coupling means being pivotally connected to a second end of each arm; and
- the first member (100) is configured to allow a longitudinal and circumferential displacement of the pivotable connector (2) along said axis (1) of the first member (100).

2. The holder of claim 1, wherein the first member comprises a guide (1IX, 11Y) for each arm to allow a longitudinal displacement of the pivotable connection(s) along said axis (1) of the first member.

3. The holder of the previous claim, wherein the guide is selected from a linear guide and a curved guide.

4. The holder according to any one of the previous claims, wherein an abutment (20) is provided which forms a touch edge for one of the arms of the second member.

5. The holder of the previous claim, wherein the abutment (20) is positioned on the first member proximal to the remote centre of motion of said object.

6. The holder of any one of the claims 4-5, wherein the abutment is mounted on a height control mechanism adapted to control the position of the abutment.

7. The holder of the previous claim, wherin the height control mechanism comprises an actuator to control the position of the abutment with respect to the holder.

8. The holder of any one of the claims 4-5, wherein the abutment is formed by a cam roller acting upon the second member (200).

9. The holder according to the previous claim, wherein the cam of the cam roller is exchangeable.

10. The holder according to any one of the previous claims wherein at least one of the arms, preferably both arms of the second member comprises a curved section at the end where it is connected to the first member.

11. The holder according to any one of the previous claims, wherein said coupling means (5) is displaced offset from said axis (1) by means of said second member (200).

12. The holder according to any one of previous claims, wherein the longitudinal displacement of the pivotable connectors (2) along said axis (1) of the first member (100) is realized by adapting a slidable position of the corresponding pivotable connectors (2) at the first member (100).

13. The holder according to any one of previous claims, wherein the circumferential displacement of the pivotable connectors (2) along said axis (1) of the first member (100) is realized by rotation of the first member along its longitudinal axis.

14. The holder of any one of the previous claims, wherein the external object (300) is a medical instrument or medical tool (e.g. cannula, trocar, ...).

15. The holder according to any one of the previous claims, wherein said holder further comprises locking means for locking the pivotable connectors and the longitudinal and circumferential displacement to fixate the position of the coupling means relative to the first member.
